# EUROPEAN PATENT SPECIFICATION

(11) **EP 2 945 618 B1**
(45) Date of publication and mention of the grant of the patent: **13.06.2018**
(21) Application number: 14702159.6
(22) Date of filing: 14.01.2014
(51) Int. Cl.: A61K 31/155, A61K 31/5383, A61P 3/10

(54) **COMBINATIONS OF A GLP1R AGONIST AND METFORMIN AND USE THEREOF FOR THE TREATMENT OF TYPE 2 DIABETES AND OTHER DISORDERS**
KOMBINATIONEN EINES GLP1R-AGONISTEN UND METFORMIN SOWIE VERWENDUNG ZUR BEHANDLUNG VON TYP-2-DIABETES UND ANDEREN ERKRANKUNGEN
COMBINAISONS D'UN AGONISTE DU GLP1R ET DE METFORMINE, ET LEUR UTILISATION POUR LE TRAITEMENT DU DIABÈTE DE TYPE 2 ET D'AUTRES TROUBLES

(30) Priority: 17.01.2013 US 201361753567 P
(43) Date of publication of application: 25.11.2015
(73) Proprietor: vTv Therapeutics LLC, High Point, NC 27265 (US)
(72) Inventor: MJALLI, Adnan M.M., Oak Ridge, North Carolina 27310 (US); CLARK, Bradley Alan, High Point, North Carolina 27265 (US); POLISETTI, Dharma Rao, High Point, North Carolina 27265 (US); QUADA, JR., James C., High Point, North Carolina 27265 (US); VALCARCE LOPEZ, Maria Carmen, Oak Ridge, North Carolina 27310 (US); ANDREWS, Robert Carl, Jamestown, North Carolina 27282 (US); DAVIS, Stephen Thomas, Durham, North Carolina 27703 (US); YOKUM, Thomas Scott, Greensboro, North Carolina 27407 (US)
(74) Representative: Cooke, Richard Spencer
(86) International application number: PCT/US2014/011394
(87) International publication number: WO 2014/113357

(56) References cited:
- WO-A1-2012/156312
- WO-A2-2009/111700
- US-A1- 2011 118 180
- US-A1- 2012 295 846

## Description

### FIELD OF INVENTION

The invention provides combinations of metformin and an agonist of the glucagon-like protein 1 receptor (GLP1R). The invention also provides for the use of a GLP1R agonist in combination with metformin for the treatment of type 2 diabetes and related disorders. The invention also provides pharmaceutical compositions comprising a GLP1R agonist and metformin.

### DESCRIPTION OF RELATED ART

Type 2 diabetes is a metabolic disorder where the disease progression may be characterized by one or more of the following: peripheral tissue insulin resistance, hyperglycemia, islet b-cell compensation, hyperinsulinemia, dyslipidemia, increased liver gluconeogenesis, or ultimate loss of beta-cell mass and function. The pathophysiological consequences of aberrant glucose and lipid metabolism are toxicity to organs such as the kidney, eye, peripheral neurons, vasculature, and heart.

Treatment of type 2 diabetes can include the administration of common agents that stimulate β-cell function or that enhance the tissue sensitivity of subjects towards insulin. Various agents are known to stimulate β-cell function, including, for example, sulfonylureas, such as tolbutamide, glibenclamide, glipizide, chlorpropamide, gliclazide, and repaglinide. Other agents are known to enhance tissue sensitivity towards insulin, such as metformin.

Although such common agents are widely used in the treatment of type 2 diabetes, the therapy often leads to unsatisfactory results. In many subjects, such treatments do not normalize blood glucose (BG) levels to a desired degree, which places subjects at a higher risk of acquiring further diabetic complications. Furthermore, these treatments are known to cause adverse effects in many subjects. For example, the sulfonylureas may induce hypoglycemia when taken alone or in combination with other drugs. And while metformin does not induce hypoglycemia to the same degree as sulfonylureas, it has other adverse effects. For example, metformin may cause gastrointestinal distress, where the incidence of such distress may increase with higher doses. Long-term use of metformin can also cause increased homocysteine levels and can lead to malabsorption of vitamin B₁₂. Metformin may also induce production of lactate, which can contribute to lactic acidosis in some patient populations.

In recent years, metformin has been approved for use in combination with other antidiabetic drugs. For example, metformin has been combined with certain sulfonylureas, including glipizide and glibenclamide. Metformin has also been combined with agents that stimulate PPAR-γ receptors, such as pioglitazone and rosiglitazone, and with agents that stimulate the release of insulin from the pancreas, such as repaglinide.

But in any combination therapy, metformin can still exhibit adverse effects, including those described above. Therefore, there is a need to discover agents that, when used with metformin, may exhibit beneficial effects related to glycemic control.

Glucagon-like peptide-1 (GLP1) is a member of the incretin family of neuroendocrine peptide hormones secreted from L-cells of the intestine in response to food ingestion. GLP1 has multiple metabolic effects that are attractive for an antidiabetic agent. A key function of GLP1 is to activate its receptor, GLP1R, on the pancreatic beta-cell to enhance glucose-dependent insulin secretion. Positive metabolic benefits of GLP1 may include, but are not limited to, suppression of excessive glucagon production, decreased food intake, delayed gastric emptying, and improvement of beta-cell mass and function. The positive effects of GLP1 on beta-cell mass and function offers the prospect that GLP1-based therapies may delay early-stage disease progression. In addition, a GLP1R agonist may also be useful in combination therapies, such as with insulin in subjects with type 1 diabetes. Unfortunately, the rapid proteolysis of GLP1 into an inactive metabolite limits its use as a therapeutic agent.

Validation of GLP1R agonists as a therapeutic modality was achieved by exendin-4 (BYETTA™, Amylin Pharmaceuticals, Inc.), a peptide GLP1 receptor agonist recently approved in some countries for the treatment of type 2 diabetes. Dosing of exendin-4 by subcutaneous administration lowers blood glucose and decreases HbA1c levels, which are important biomarker measurements for disease control. Thus, an oral GLP1 receptor agonist should provide glycemic control while offering the convenience of oral dosing.

The GLP1 receptor (GLP1R) belongs to the class B receptor sub-class of the G protein-coupled receptor (GPCR) superfamily that regulates important physiological and pathophysiological processes. In addition to the seven transmembrane domains characteristic of all GPCR family members, class B GPCRs contain a relatively large N-terminal domain. It is believed that the binding and activation of these receptors by large natural peptide ligands require both the N-terminal domain and the transmembrane domain of the receptor. The identification of low-molecular-weight non-peptide molecules that bind and activate class B GPCRs has proven to be difficult.

Because peptides, such as GLP1, may lack sufficient oral bioavailability for consideration as oral drug agents, small molecule modulators of GLP1R with oral bioavailability are desired. WO 2009/111700 and WO 2010/114824 describe various small-molecule GLP1R agonists.

### BRIEF SUMMARY OF THE INVENTION

The invention provides combinations of metformin and an agonist of the glucagon-like protein type 1 receptor (GLP1R) that may be useful for the treatment of type 2 diabetes and other disorders. In an embodiment, the GLP1R agonist is orally bioavailable.

In one aspect, the invention provides pharmaceutical compositions comprising an oral GLP1R agonist and metformin. In some embodiments, the pharmaceutical composition also comprises a pharmaceutically acceptable carrier. In some such embodiments, the pharmaceutical composition may further comprise one or more other additional substances, such as pharmaceutically acceptable excipients, diluents, and the like. In some embodiments the pharmaceutical composition is a solid-state composition. In other embodiments, the pharmaceutical composition is a liquid composition. In other embodiments, the pharmaceutical composition is an oral pharmaceutical composition.

In another aspect, the invention provides methods of treating type 2 diabetes by administering to a subject a GLP1R agonist in combination with metformin. In an embodiment, the GLP1R agonist is orally bioavailable. In some embodiments, the GLP1R agonist and metformin are administered simultaneously, either in separate dosage forms or the same dosage form. In other embodiments, the GLP1R agonist and metformin are not administered simultaneously, but are instead administered according to a sequence. In other embodiments, either the GLP1R agonist or metformin is administered subsequent to the other, so that an amount of both are simultaneously present in the subject (as determined, for example, by analysis of the subject's blood or blood plasma).

In another aspect, the invention provides methods of lowering blood glucose in a subject by administering to the subject a GLP1R agonist in combination with metformin. In an embodiment, the GLP1R agonist is orally bioavailable. In some embodiments, the GLP1R agonist and metformin are administered simultaneously, either in separate dosage forms or the same dosage form. In other embodiments, the oral GLP1R agonist and metformin are not administered simultaneously, but are instead administered according to a sequence. In other embodiments, either the GLP1R agonist or metformin is administered subsequent to the other, so that an amount of both are simultaneously present in the subject (as determined, for example, by analysis of the subject's blood or blood plasma).

Additional features and aspects of the present invention are described hereinafter.

### BRIEF DESCRIPTION OF DRAWINGS

Not applicable.

### DETAILED DESCRIPTION

As used throughout this specification, the term "OAD1" refers to (S)-3-(4'-cyanobiphenyl-4-yl)-2- {[(3R,7S)-3-[4-(3,4-dichloro-benzyloxy)-phenyl]-1-methyl-2-oxo-6-((S)-1-phenyl-propyl)-2,3,5,6,7,8-hexahydro-1*H*-4-oxa-1,6-diaza-anthracene-7-carbonyl]-amino}-propionic acid.

As used throughout this specification, the term "OAD2" refers to (S)-2-{[(3S,8S)-3-[4-(3,4-dichloro-benzyloxy)-phenyl]-7-((S)-1-phenyl-propyl)-2,3,6,7,8,9-hexahydro-[1,4]dioxino[2,3-g]isoquinoline-8-carbonyl]-amino}-3-[4-(2,3-dimethyl-pyridin-4-yl)-phenyl]-propionic acid.

In the early stages, subjects with type 2 diabetes may exhibit a decreasing ability of their pancreas to secrete sufficient insulin to control postprandial blood-glucose levels. At first, type 2 diabetics may be able to control progression of the disease by following dietary restrictions, such as consuming foods having a low glycemic index. But as the disease progresses, diet alone is insufficient to control blood-glucose levels. Thus, medical intervention becomes necessary. At this stage (or even in advance of this stage), physicians may prescribe an oral antidiabetic agent to aid in glycemic control. Common oral antidiabetic agents include, for example, sulfonylureas, such as glibenclamide, and biguanides, such as metformin.

These common antidiabetics may eventually have undesirable side-effects in certain patient populations, and may also eventually fail to provide desirable levels of glycemic control. Thus, scientists have continued to search for compounds that can replace or supplement the use of these common antidiabetics. Agonists of the glucagon-like peptide type 1 receptor (GLP1R agonists) represent one such class of compounds.

Various GLP1R agonists are known. For example, the preparation and pharmaceutical use of OAD1 and pharmaceutically acceptable salts thereof is described in WO 2009/111700.

OAD1 or a pharmaceutically acceptable salt thereof may have poor aqueous solubility. For example, the aqueous solubility of the hydrochloric acid salt of OAD1 increases at pH levels greater than 7 but it is less than 0.01 mg/mL at pH 7 or at a pH of less than 7. This poor aqueous solubility at pH between 6 and 7 may correspond to poor absorption or poor pharmacokinetics for OAD1 hydrochloride when administered orally. Thus, there is a need to provide an oral dosage composition of OAD1 or a pharmaceutically acceptable salt thereof with improved dissolution and/or absorption of OAD1 or a pharmaceutically acceptable salt thereof leading to improved oral bioavailability. Use of metal salts of an acid that demonstrates basic properties (such as alkali metal carbonates) to increase the bioavailability of an oral dosage composition of a GLP1R agonist other than OAD1 is provided in US Patent Publication No. 2011/0064806. However, these metal salts, such as sodium carbonate and/or sodium bicarbonate, were in some instances used in a weight to weight ratio of 2:1 relative to the GLP1R agonist. See Example 19 in US Pub. No. 2011/0064806.

Thus, the invention provides pharmaceutical compositions comprising a GLP1R agonist and metformin and methods comprising administering to a subject a GLP1R agonist in combination with metformin. The pharmaceutical compositions and methods of treatment comprising a GLP1R agonist and metformin may have improved dissolution, absorption, oral exposure, and/or bioavailability profiles in a subject for the GLP1R agonist as compared to pharmaceutical compositions and methods of treatment that do not include metformin. Alternatively, the pharmaceutical compositions and/or methods of treatment comprising a GLP1R agonist and metformin may improve the therapeutic effectiveness of metformin or the GLP1R agonist in a subject as compared to the same doses of either metformin or the GLP1R agonist as a monotherapy.

### Metformin

N,N-dimethylimidodicarbonimidic diamide is often referred to as metformin or 1,1-dimethylbiguanide. Metformin can exist as a free base, or may form salts, including pharmaceutically acceptable salts, such as a hydrochloride salt (e.g., a mono-hydrochloride salt). See Remington's, 21st edition, pp. 1454-55 (2006). As used herein, the term "metformin" is not limited to the free base, but also includes metformin salts, such as pharmaceutically acceptable salts of metformin, hydrochloride salts of metformin, and a mono-hydrochloride salt. In an embodiment, the metformin may be metformin hydrochloride salt. As used herein, the term "1,1-dimethylbiguanide" refers only to the free base unless the text expressly indicates that salted forms are also contemplated.

The metformin may be included in any suitable dosage form. For example, metformin may exist in a powder, a tablet, a capsule, and the like. Such dosage forms may, in some embodiments, also include specialized coatings, matrices, and the like to give a sustained release, a controlled release, enteric release, etc. In some embodiments, metformin may exist in a dosage form with an oral GLP1R agonist. In some embodiments, the oral GLP1R agonist is OAD1, OAD2, or a pharmaceutically acceptable salt thereof.

As used throughout this specification, the term "pharmaceutically acceptable salt" refers to salts of a free acid or a free base which are not biologically undesirable and are generally prepared by reacting the free base with a suitable organic or inorganic acid or by reacting the acid with a suitable organic or inorganic base. The term may be used in reference to any compound, including 1,1-dimethylbiguanide, and a GLP1R agonist (having a free acid or free base functionality), etc. Representative salts include the following salts: Acetate, Benzenesulfonate, Benzoate, Bicarbonate, Bisulfate, Bitartrate, Borate, Bromide, Calcium Edetate, Camsylate, Carbonate, Chloride, Clavulanate, Citrate, Dihydrochloride, Edetate, Edisylate, Estolate, Esylate, Fumarate, Gluceptate, Gluconate, Glutamate, Glycollylarsanilate, Hexylresorcinate, Hydrabamine, Hydrobromide, Hydrochloride, Hydroxynaphthoate, Iodide, Isethionate, Lactate, Lactobionate, Laurate, Malate, Maleate, Mandelate, Mesylate, Methylbromide, Methylnitrate, Methylsulfate, Monopotassium Maleate, Mucate, Napsylate, Nitrate, N-methylglucamine, Oxalate, Pamoate (Embonate), Palmitate, Pantothenate, Phosphate/diphosphate, Polygalacturonate, Potassium, Salicylate, Sodium, Stearate, Subacetate, Succinate, Tannate, Tartrate, Teoclate, Tosylate, Triethiodide, Trimethylammonium and Valerate. When an acidic substituent is present (e.g., in a GLP1R agonist), such as -COOH, there can be formed the ammonium, morpholinium, sodium, potassium, barium, calcium salt, and the like, for use as the dosage form. When a basic group is present (e.g., in a GLP1R agonist or 1,1-dimethylbiguanide), such as amino or a basic heteroaryl radical, such as pyridyl, an acidic salt can form, such as hydrochloride, hydrobromide, phosphate, sulfate, trifluoroacetate, trichloroacetate, acetate, oxalate, maleate, pyruvate, malonate, succinate, citrate, tartarate, fumarate, mandelate, benzoate, cinnamate, methanesulfonate, ethanesulfonate, picrate and the like, and include acids related to the pharmaceutically-acceptable salts listed in Stephen M. Berge, et al., Journal of Pharmaceutical Science, Vol. 66, pp. 1-19 (1977).

### GLP1R Agonists

As used throughout this specification, a "GLP1R agonist" is a compound that binds to a cellular GLP1 receptor and elicits stimulation of the adenylate cyclase pathway resulting in increased synthesis of cyclic AMP and release of insulin if the cell is a mammalian pancreatic beta cell. WO 2009/111700 and WO 2010/114824 each provide a non-limiting list of compounds that are GLP1R agonists. In some embodiments, the GLP1R agonist is a small molecule, such as a molecule having a molecular weight between 200 amu and 2000 amu, or between 200 amu and 1200 amu, or between 500 amu and 1000 amu. In some embodiments, the GLP1R agonist is orally bioavailable (referred to as an "oral GLP1R agonist"). In some embodiments, the oral GLP1R agonist has an absolute bioavailability after oral administration to a subject of at least 1%, or at least 2%, or at least 3%, or at least 4%, or at least 5%, or at least 6%, or at least 7%, or at least 8%, or at least 9%, or at least 10%, or at least 11%, or at least 12%, or at least 13%, or at least 14%, or at least 15%, or at least 16%, or at least 17%, or at least 18%, or at least 19%.

As used throughout this specification, the conjunction "or," when used to define a group, defines the group in such a way that one or more of the group members is present. Thus, the phrase "A, B, or C" includes the following non-limiting embodiments: A is present, but B and C are not; A and B are present, but C is not; A, B, and C are all present. Thus, the presence of one member of the group does not necessarily imply the absence of any others. In contrast, the conjunction "and," when used to define a group, implies that at least each of the recited members of the group is present.

The pharmaceutical compositions and/or the methods of treatment of the invention may exhibit improved dissolution, absorption, oral exposure, and/or bioavailability of the GLP1R agonist (such as OAD1, OAD2, or pharmaceutically acceptable salts thereof) upon administration to a subject relative to pharmaceutical compositions and/or the methods of treatment that do not include metformin. Alternatively, the pharmaceutical compositions and/or methods of treatment comprising a GLP1R agonist and metformin may improve the therapeutic effectiveness of metformin or the GLP1R agonist in a subject as compared to the same doses of either metformin or the GLP1R agonist as a monotherapy.

As used herein, the term "improved bioavailability" means that the bioavailability of the GLP1R agonist is increased relative to the bioavailability of the GLP1R agonist in the absence of metformin. For example, the improvement in bioavailability of the GLP1R agonist may be at least two times, at least three times, at least five times, or at least ten times that of the GLP1R agonist in the absence of metformin. It is within the ability of one of skill in the art to determine the bioavailability of a compound or composition using methods generally accepted in the art. For example, the maximum concentration (Cₘₐₓ) of OAD1, OAD2, or a pharmaceutically acceptable salt thereof in plasma or the overall amount of OAD1, OAD2, or a pharmaceutically acceptable salt thereof in plasma after a dosage, e.g., area-under-the-curve (AUC), may be used for the comparison. These pharmacokinetic measurements may be determined by conventional techniques. For example, in various embodiments, the concentration in plasma may be determined by a LC-MS/MS assay following a protein precipitation step with acetonitrile. In additional embodiments, pharmacokinetic analysis may be performed using the WinNonlin™ software program, which is available from Pharsight, Inc. of Mountain View, California, USA. The area under the plasma concentration-time curve (AUC₀₋ₜ) may be calculated from the first time point (0 min) up to the last time point with measurable drug concentration. The AUC_{0-inf} may be calculated as the sum of AUC₀₋ₜ and Cpred/λz, where Cpred was the predicted concentration at the time of the last quantifiable concentration.

### Combination Therapy

In some embodiments of the invention, metformin is administered in combination with a GLP1R agonist, such as in combination with OAD1, OAD2, or a pharmaceutically acceptable salt thereof. In an embodiment, the GLP1R agonist is orally bioavailable. Administration is typically to a subject, such as a human, for the treatment of a disease, disorder, or condition.

As used herein, "administer" or "administering" means to introduce, such as to introduce to a subject a compound or composition. The term is not limited to any specific mode of delivery, and may include, for example, subcutaneous delivery, intravenous delivery, intramuscular delivery, intracisternal delivery, delivery by infusion techniques, transdermal delivery, oral delivery, nasal delivery, and rectal delivery. Furthermore, depending on the mode of delivery, the administering can be carried out by various individuals, including, for example, a health-care professional (e.g., physician, nurse, etc.), a pharmacist, or the subject (i.e., self-administration).

As used herein, "treat" or "treating" or "treatment" can refer to one or more of: delaying the progress of a disease, disorder, or condition; controlling a disease, disorder, or condition; delaying the onset of a disease, disorder, or condition; ameliorating one or more symptoms characteristic of a disease, disorder, or condition; or delaying the recurrence of a disease, disorder, or condition, or characteristic symptoms thereof, depending on the nature of the disease, disorder, or condition and its characteristic symptoms.

As used herein, "subject" refers to any mammal such as, but not limited to, humans, horses, cows, sheep, pigs, mice, rats, dogs, cats, and primates such as chimpanzees, gorillas, and rhesus monkeys. In an embodiment, the "subject" is a human. In another embodiment, the "subject" is a human who exhibits one or more symptoms characteristic of a disease, disorder, or condition. In another embodiment, the "subject" is a human who has a disease, disorder, or condition in which GLP1 receptor is involved. The term "subject" does not require one to have any particular status with respect to a hospital, clinic, or research facility (e.g., as an admitted patient, a study participant, or the like).

As used herein, the term "in combination with," when used, for example, in the context of administering a compound in combination with another compound, places no limit on the method, mode, form, etc. of the administration, so long as the administration results in both compounds being simultaneously biologically available to a subject (e.g., present in the blood plasma) at a common point in time.

As noted above, in some embodiments, metformin is administered in combination with a GLP1R agonist, such as in combination with OAD1, OAD2, or a pharmaceutically acceptable salt thereof. In some such embodiments, metformin and a GLP1R agonist are administered simultaneously, for example, via oral administration. For example, metformin and a GLP1R agonist are delivered in a common dosage form, where the dosage form comprises both metformin and a GLP1R agonist, such as OAD1, OAD2, or a pharmaceutically acceptable salt thereof. In another example, metformin and a GLP1R agonist are delivered in two or more separate dosage forms that are administered at approximately the same time (e.g., within less than 30 minutes, or within less than 15 minutes, or within less than 10 minutes, or within less than 5 minutes, or within less than 2 minutes of each other), where a first dosage form comprises metformin and a second dosage form comprises a GLP1R agonist, such as OAD1 or OAD2. In further such embodiments, metformin and a GLP1R agonist are administered sequentially, preferably via oral administration. For example, metformin or the GLP1R agonist can be administered about 30 minutes apart, or about 1 hour apart, or about 2 hours apart, or about 4 hours apart, or about 8 hours apart, or about 12 hours apart, where metformin is administered earlier than the GLP1R agonist, or vice versa. In even further such embodiments, either metformin or the GLP1R agonist is administered subsequent to the other, so long as the administration results in both compounds being simultaneously biologically available to a subject (e.g., present in the blood plasma) at a common point in time. For example, one can be administered about 30 minutes after, or about 1 hour after, or about 2 hours after, or about 4 hours after, or about 8 hours after, or about 12 hours after the administration of the other.

### Pharmaceutical Compositions

In some embodiments of the invention, metformin and/or a GLP1R agonist may be included within a pharmaceutical composition. In some such embodiments, a single pharmaceutical composition comprises both metformin and a GLP1R agonist. In a further embodiment, a single pharmaceutical composition comprising both metformin and a GLP1R agonist may be an oral pharmaceutical composition. In some further such embodiments, two or more pharmaceutical compositions are provided, where at least one pharmaceutical composition comprises metformin and at least one other pharmaceutical composition comprises a GLP1R agonist.

As used herein, the term "pharmaceutical composition" refers to a composition (e.g., a granulated powder or a liquid) that contains a pharmaceutically active ingredient (e.g., metformin and/or a GLP1R agonist) and a pharmaceutically acceptable carrier. As used herein, the term "pharmaceutically acceptable" refers to a substance that is not generally biologically undesirable at the administered quantities. In some embodiments, metformin and the GLP1R agonist are included in separate pharmaceutical compositions, each of which also includes a pharmaceutically acceptable carrier. In other embodiments, metformin and the GLP1R agonist are included in the same pharmaceutical composition, which also includes a pharmaceutically acceptable carrier. In a further embodiment, a single pharmaceutical composition comprising both metformin and a GLP1R agonist may be an oral pharmaceutical composition and the GLP1R agonist may have a molecular weight between 200 and 2000 amu.

### Dosage Forms

The pharmaceutical compositions, described herein, can be packaged in a form for oral administration as discrete units (i.e., dosage forms), such as capsules, tablets, sachets, or the like. Preparation of the solid compositions in forms intended for oral administration is within the ability of one skilled in the art, including the selection of pharmaceutically acceptable additional ingredients from the groups listed above in order to provide pharmaceutically elegant and palatable preparations. Such pharmaceutical compositions may be prepared by methods known in the pharmaceutical formulation art, for example, see Remington's Pharmaceutical Sciences, 18th Ed., (Mack Publishing Company, Easton, Pa., 1990).

In some embodiments, administration of metformin in combination with a GLP1R agonist can include administration of a dosage form that comprises metformin and a GLP1R agonist, such as OAD1, OAD2, or a pharmaceutically acceptable salt thereof. Such dosage forms may contain any suitable amounts of OAD1, OAD2, or a pharmaceutically acceptable salt thereof and metformin, so long as the resulting can be readily administered orally. In some embodiments, the dosage form may comprise from 1 mg to 1000 mg, or from 25 mg to 800 mg, or from 50 mg to 750 mg, or from 75 mg to 600 mg, or from 100 mg to 400 mg, or from 150 mg to 300 mg, or from 1 mg to 200 mg, or from 1 mg to 150 mg, or from 1 mg to 100 mg, or from 1 mg to 50 mg, or from 10 mg to 50 mg, of from 25 mg to 75 mg, or from 50 mg to 100 mg, or from 75 mg to 125 mg, or from 100 mg to 150 mg, or from 125 mg to 175 mg, or from 150 mg to 200 mg, or from 175 mg to 225 mg of a GLP1R agonist, such as OAD1, OAD2, or a pharmaceutically acceptable salt thereof. In any of these embodiments, the dosage form may also comprise from 10 mg to 1000 mg, or from 100 mg to 1000 mg, or from 200 mg to 800 mg, or from 400 mg to 800 mg of metformin. In a further embodiment, the dosage form may comprise metformin hydrochloride. In a further embodiment, the dosage form may comprise 250 mg, 500 mg, 850 mg, or 1000 mg of metformin hydrochloride.

In some further embodiments, administration of metformin in combination with a GLP1R agonist can include administration of two or more dosage forms, where a first dosage form comprises metformin and a second dosage form comprises a GLP1R agonist, such as OAD1, OAD2, or a pharmaceutically acceptable salt thereof. In some embodiments, the second dosage form may comprise from 1 mg to 1000 mg, or from 25 mg to 800 mg, or from 50 mg to 750 mg, or from 75 mg to 600 mg, or from 100 mg to 400 mg, or from 150 mg to 300 mg, or from 1 mg to 200 mg, or from 1 mg to 150 mg, or from 1 mg to 100 mg, or from 1 mg to 50 mg, or from 10 mg to 50 mg, of from 25 mg to 75 mg, or from 50 mg to 100 mg, or from 75 mg to 125 mg, or from 100 mg to 150 mg, or from 125 mg to 175 mg, or from 150 mg to 200 mg, or from 175 mg to 225 mg of a GLP1R agonist, such as OAD1, OAD2, or a pharmaceutically acceptable salt thereof. In any of these embodiments, the first dosage form may comprise from 10 mg to 1000 mg, or from 100 mg to 1000 mg, or from 200 mg to 800 mg, or from 400 mg to 800 mg of metformin. In a further embodiment, the dosage form may comprise metformin hydrochloride. In a further embodiment, the dosage form may comprise 250 mg, 500 mg, 850 mg, or 1000 mg of metformin hydrochloride.

### Dosage Quantities

In embodiments of the invention, an amount of a GLP1R agonist, such as OAD1, OAD2, or a pharmaceutically acceptable salt thereof, is administered to a subject (e.g., a human) in combination with metformin. The amount of the GLP1R agonist administered can vary depending on various factors, including but not limited to, the weight of the subject, the nature and/or extent of the subject's disease, etc. In some embodiments, a GLP1R agonist, such as OAD1, OAD2, or a pharmaceutically acceptable salt thereof, is administered to a subject (e.g., a human), in combination with metformin, in an amount that ranges from 1 mg/day to 1000 mg/day, or from 25 mg/day to 800 mg/day, or from 50 mg/day to 750 mg/day, or from 75 mg/day to 600 mg/day, or from 100 mg/day to 400 mg/day, or from 1 mg to 200 mg/day, or from 1 mg to 150 mg/day, or from 1 mg to 100 mg/day, or from 1 mg to 50 mg/day, or from 10 mg to 50 mg/day, of from 25 mg to 75 mg/day, or from 50 mg to 100 mg/day, or from 75 mg to 125 mg/day, or from 100 mg to 150 mg/day, or from 125 mg to 175 mg/day, or from 150 mg to 200 mg/day, or from 175 mg to 225 mg/day of a GLP1R agonist. In some further embodiments, a GLP1R agonist, such as OAD1, OAD2, or a pharmaceutically acceptable salt thereof, is administered to a subject (e.g., a human), in combination with metformin, in an amount of about 100 mg/day, or about 200 mg/day, or about 300 mg/day, or about 250 mg/day, or about 400 mg/day, or about 500 mg/day, or about 600 mg/day, or about 700 mg/day, or about 800 mg/day, or about 850 mg/day, or about 1000 mg/day of metformin, or between 100 mg to 3000 mg/day. In even some further embodiments, a GLP1R agonist, such as OAD1, OAD2, or a pharmaceutically acceptable salt thereof, is administered to a subject (e.g., a human), in combination with metformin. In each of these embodiments, the GLP1R agonist, such as OAD1, OAD2, or a pharmaceutically acceptable salt thereof, may be administered orally.

As used herein, the term "therapeutically effective amount" refers to an amount of an active ingredient (e.g., metformin or GLP1R agonist, such as OAD1, OAD2, or pharmaceutically acceptable salt thereof) that elicits the biological or medicinal response in a tissue, system, or subject that is being sought by a researcher, veterinarian, medical doctor, patient or other clinician, which includes reduction or alleviation of the symptoms of the disease being treated.

As a monotherapy, metformin may be administered to human subjects in amounts between 1000 mg/day and 2500 mg/day. See Remington's, 21st edition, pp. 1454-55 (2006). In smaller doses, metformin may offer negligible therapeutic benefits when administered as a monotherapy. Id. at 1455.

In embodiments of the invention, a GLP1R agonist, such as OAD1, OAD2, or a pharmaceutically acceptable salt thereof, is administered to a subject (e.g., a human) in combination with an amount of metformin. In some embodiments, the amount of metformin administered to a subject (e.g., a human) ranges from 250 mg/day to 2500 mg/day, or from 350 mg/day to 2000 mg/day, or from 400 mg/day to 1500 mg/day, or from 1000 mg/day to 2500 mg/day. In some further embodiments, the amount of metformin administered to a subject (e.g., a human) is about 1000 mg/day, or about 1250 mg/day, or about 1500 mg/day, or about 1750 mg/day, or about 2000 mg/day, or about 2250 mg/day, or about 2500 mg/day.

In some embodiments of the invention, a GLP1R agonist, such as OAD1, OAD2, or a pharmaceutically acceptable salt thereof, is administered to a subject (e.g., a human) in combination with a suboptimal amount of metformin. As used herein in reference to metformin, a "suboptimal amount" is an amount that is less than a therapeutically effective amount as a monotherapy in a typical subject (e.g., a human subject, or a human subject who suffers from type 2 diabetes, or a human subject in need of glycemic control). In some such embodiments, the suboptimal amount of metformin is an amount that ranges from 0.01 mg/day to 1000 mg/day, or from 10 mg/day to 850 mg/day, or from 37 mg/day to 750 mg/day, or from 50 mg/day to 700 mg/day, or from 75 mg/day to 600 mg/day, or from 100 mg/day to 500 mg/day. In some embodiments of the invention, a GLP1R agonist, such as OAD1, OAD2, or a pharmaceutically acceptable salt thereof, is administered to a subject (e.g., a human) in combination with metformin, wherein the amount of the GLP1R agonist is suboptimal. As used herein in reference to the GLP1R agonist, a "suboptimal amount" is an amount that is less than a therapeutically effective amount as a monotherapy in a typical subject (e.g., a human subject, or a human subject who suffers from type 2 diabetes, or a human subject in need of glycemic control). In some such embodiments, the suboptimal amount of the GLP1R agonist is an amount that is less than 250 mg/day, less than 200 mg/day, less than 150 mg/day, less than 100 mg/day, less than 50 mg/day, less than 25 mg/day, less than 10 mg/day of a GLP1R agonist, such as OAD1, OAD2, or a pharmaceutically acceptable salt thereof. In another embodiment, the suboptimal amount of the GLP1R agonist is an amount that is between 1 mg/day and 250 mg/day, or between 5 mg/day and 200 mg/day, or between 10 mg/day and 150 mg/day, or between 15 mg/day and 100 mg/day, or between 20 mg/day and 50 mg/day, or between 1 mg/day and 200 mg/day, or between 1 mg/day and 150 mg/day, or between 1 mg/day and 100 mg/day, or between 1 mg/day and 50 mg/day, or between 10 mg/day and 50 mg/day, or between 25 mg/day and 75 mg/day, or between 50 mg/day and 100 mg/day, or between 75 mg/day and 125 mg/day, or between 100 mg/day and 150 mg/day, or between 125 mg/day and 175 mg/day, or between 150 mg/day and 200 mg/day, or between 175 mg/day and 225 mg/day of a GLP1R agonist, such as OAD1, OAD2, or a pharmaceutically acceptable salt thereof.

### Pharmaceutical Compositions Containing Metformin and a GLP1R agonist

In a further aspect of the invention, a GLP1R agonist, such as OAD1, OAD2, or a pharmaceutically acceptable salt thereof, can be included in a pharmaceutical composition with metformin. In some embodiments, the pharmaceutical composition is a solid. In other embodiments, the pharmaceutical composition is a liquid. In other embodiments, the pharmaceutical composition is a suspension.

In some embodiments, the GLP1R agonist and the metformin are intermixed, optionally in the presence of a pharmaceutically acceptable carrier, such that the GLP1R agonist and metformin are homogeneously distributed throughout the composition. In other embodiments, a pharmaceutical composition comprises both a GLP1R agonist and metformin, and optionally a pharmaceutically acceptable carrier, where the GLP1R agonist and the metformin are not homogeneously distributed throughout the composition. As a non-limiting example, the composition may be a solid composition that comprises a bi-layered granule, where one layer in the granule is enriched in a GLP1R agonist and the other layer in the granule is enriched in metformin.

Such granules can be made by any suitable granulation method known in the art, including but not limited to, various dry granulation and wet granulation techniques. Furthermore, the particle size and the distribution of particle sizes of the granules can be adjusted according to known techniques to achieve release profiles, dissolution, and the like. In some embodiments, the invention provides a pharmaceutical composition that comprises granules that each comprise a GLP1R agonist and metformin. In some such embodiments, at least 80%, or at least 85%, or at least 90%, or at least 95% (by weight) of said granules have a particle size that is between 1 µm and 1 mm. Further, in some such embodiments, at least 80%, or at least 85%, or at least 90%, or at least 95% (by weight) of said granules have a particle size that is between 1 µm and 500 µm.

In an embodiment, the metformin may be in the form of a pharmaceutically acceptable salt. In a further embodiment, the metformin may be in the form the hydrochloride salt. In a separate embodiment, the metformin may be in the form of 1,1-dimethylbiguanide (i.e., the free base).

In an embodiment, the invention provides a pharmaceutical composition comprising a GLP1R agonist, such as OAD1, OAD2, or a pharmaceutically acceptable salt thereof, and metformin, the amount of GLP1R agonist is a suboptimal amount. In an embodiment, the suboptimal amount of the GLP1R agonist is less than 250 mg, less than 200 mg, less than 150 mg, less than 100 mg, less than 50 mg, less than 25 mg, or less than 10 mg. In another embodiment, the suboptimal amount of the GLP1R agonist is between 1 mg and 250 mg, or between 5 mg and 200 mg, or between 10 mg and 150 mg, or between 15 mg and 100 mg, or between 20 mg and 50 mg, or between 1 mg and 200 mg, or between 1 mg and 150 mg, or between 1 mg and 100 mg, or between 1 mg and 50 mg, or between 10 mg and 50 mg, or between 25 mg and 75 mg, or between 50 mg and 100 mg, or between 75 mg and 125 mg, or between 100 mg and 150 mg, or between 125 mg and 175 mg, or between 150 mg and 200 mg, or between 175 mg and 225 mg of a GLP1R agonist, such as OAD1, OAD2, or a pharmaceutically acceptable salt thereof. In a further embodiment, the amount of metformin in the pharmaceutical composition is between 100 mg and 1000 mg, or between 100 mg and 900 mg, or between 100 mg and 800 mg, or between 100 mg and 700 mg, or between 100 mg and 600 mg, or between 100 mg and 500 mg, or between 100 mg and 400 mg, or between 50 mg and 150 mg, or between 100 mg and 200 mg, or between 150 mg and 250 mg, or between 200 mg and 300 mg, or between 250 mg and 350 mg, or between 300 mg and 400 mg, or between 350 mg and 450 mg, or between 400 mg and 500 mg, or between 450 mg and 550 mg, or between 500 mg and 600 mg, or between 550 mg and 650 mg, or between 600 mg and 700 mg, or between 650 mg and 750 mg, or between 700 mg and 800 mg, or between 750 mg and 850 mg, or between 800 mg and 900 mg, or between 850 mg and 950 mg, or between 900 mg and 1000 mg. In a further embodiment, the weight to weight ratio of the suboptimal amount of the GLP1 agonist to metformin is 1:2 or greater, for example between 1:2 and 1:100, or between 1:2 and 1:50, or between 1:2 and 1:25, or between 1:2 and 1:20, or between 1:2 and 1:10, or between 1:3 and 1:50, or between 1:3 and 1:25, or between 1:3 and 1:10, or between 1:4 and 1:50, or between 1:4 and 1:25, or between 1:4 and 1:10, or between 1:5 and 1:50, or between 1:5 and 1:25, or between 1:5 and 1:10, or between 1:3 and 1:6. Thus, for example, the invention provides a pharmaceutical composition comprising OAD1, OAD2, or a pharmaceutically acceptable salt thereof and metformin, wherein the OAD1, OAD2, or a pharmaceutically acceptable salt thereof is in an amount less than 250 mg, wherein the amount of metformin is between 100 mg and 1000 mg, and wherein the weight to weight ratio of OAD1, OAD2, or a pharmaceutically acceptable salt thereof to metformin is between 1:2 and 1:100, as well as all possible subcombinations provided in this paragraph or in any of the embodiment contained herein. In a further embodiment, the molar ratio of the suboptimal amount of the GLP1 agonist to metformin is about 1:1, for example between 1:0.5 and 1:2, or between 1:0.9 and 1:1.1. Thus, for example, the invention provides a pharmaceutical composition comprising OAD1, OAD2, or a pharmaceutically acceptable salt thereof and metformin, wherein the OAD1, OAD2, or a pharmaceutically acceptable salt thereof is in an amount less than 250 mg, wherein the amount of metformin is between 100 mg and 1000 mg, and wherein the molar ratio of OAD1, OAD2, or a pharmaceutically acceptable salt thereof and metformin is between 1:0.9 and 1:1.1. In a further embodiment, the metformin is metformin hydrochloride. In a further embodiment, the metformin is 1,1-dimethylbiguanide.

In another embodiment, the invention provides a pharmaceutical composition comprising a GLP1R agonist, such as OAD1, OAD2, or a pharmaceutically acceptable salt thereof, and metformin, wherein the amount of the metformin is a suboptimal amount.

In another embodiment, the invention provides a pharmaceutical composition comprising a GLP1R agonist, such as OAD1, OAD2, or a pharmaceutically acceptable salt thereof, and metformin, wherein the amount of the GLP1 agonist and metformin are suboptimal amounts.

In any embodiment where metformin and/or a GLP1R agonist are included in a pharmaceutical composition, such pharmaceutical compositions may be in a form suitable for oral use, for example, as tablets, troches, lozenges, aqueous, or oily suspensions, dispersible powders or granules, emulsions, hard or soft capsules, or syrups or elixirs. Compositions intended for oral use may be prepared according to any known method, and such compositions may contain one or more agents selected from the group consisting of sweetening agents, flavoring agents, coloring agents, and preserving agents in order to provide pharmaceutically elegant and palatable preparations. Tablets may contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch or alginic acid; binding agents, for example, starch, gelatin or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques to form osmotic therapeutic tablets for controlled release.

### Methods of Treatment

In another aspect, the invention provides methods of treating type 2 diabetes by administering to a subject a GLP1R agonist, such as OAD1, OAD2, or a pharmaceutically acceptable salt thereof, in combination with metformin, according to any of the embodiments described in the foregoing sections.

In another aspect, the invention provides methods of lowering blood-glucose in a subject by administering to the subject a GLP1R agonist, such as OAD1, OAD2, or a pharmaceutically acceptable salt thereof, in combination with metformin, according to any of the embodiments described in the foregoing sections.

In another aspect, the invention provides methods of increasing the therapeutic effectiveness (in terms of enhanced glucose-lowering effect) of metformin by administering to a subject a GLP1R agonist, such as OAD1, OAD2, or a pharmaceutically acceptable salt thereof, in combination with metformin, according to any of the embodiments described in the foregoing sections.

In another aspect, the invention provides methods of increasing the therapeutic effectiveness (in terms of enhanced glucose-lowering effect) of a GLP1R 1 agonist, such as OAD1, OAD2, or a pharmaceutically acceptable salt thereof, by administering to a subject metformin in combination with the GLP1R agonist, according to any of the embodiments described in the foregoing sections.

In another aspect, the invention provides methods of increasing the oral bioavailability or oral exposure of a GLP1R 1 agonist, such as OAD1, OAD2, or a pharmaceutically acceptable salt thereof, by administering to a subject metformin in combination with the GLP1R agonist, according to any of the embodiments described in the foregoing sections.

In another aspect, the invention provides methods of reducing the amount of active pharmaceutical ingredients administered to a subject while still achieving similar or improved therapeutic effectiveness comprising administering to a subject a GLP1R 1 agonist, such as OAD1, OAD2, or a pharmaceutically acceptable salt thereof, in combination with metformin. The reduction of the amount of active pharmaceutical ingredients may be relative to either or both active pharmaceutical ingredients as a monotherapy.

In another aspect, the invention provides methods of treating a condition comprising administering to a subject a GLP1R agonist, such as OAD1, OAD2, or a pharmaceutically acceptable salt thereof, in combination with metformin, according to any of the embodiments described in the foregoing sections, wherein the condition is selected from metabolic syndrome, glucose intolerance, hyperglycemia, dyslipidemia, hypertriglyceridemia, syndrome X, insulin resistance, impaired glucose tolerance (IGT), obesity, diabetic dyslipidemia, hyperlipidemia, arteriosclerosis, atherosclerosis, other cardiovascular diseases, hypertension, metabolic disorders where agonism of GLP1R is beneficial, or complications resulting from or associated with diabetes, including, but not limited to, neuropathy, retinopathy, nephropathy, and impaired wound healing.

In another aspect, the invention provides methods of treating type 1 diabetes by administering to a subject a GLP1R agonist, such as OAD1, OAD2, or a pharmaceutically acceptable salt thereof, in combination with metformin, according to any of the embodiments described in the foregoing sections.

In another aspect, the invention provides methods of treating obesity by administering to a subject a GLP1R agonist, such as OAD1, OAD2, or a pharmaceutically acceptable salt thereof, in combination with metformin, according to any of the embodiments described in the foregoing sections.

In another aspect, the invention provides methods of slowing gastric emptying by administering to a subject a GLP1R agonist, such as OAD1, OAD2, or a pharmaceutically acceptable salt thereof, in combination with metformin, according to any of the embodiments described in the foregoing sections.

In another aspect, the invention provides methods of lowering an HbA1c level by administering to a subject a GLP1R agonist, such as OAD1, OAD2, or a pharmaceutically acceptable salt thereof, in combination with metformin, according to any of the embodiments described in the foregoing sections. In an embodiment, the method may reduce the amount of HbAlC in a subject in need thereof by at least 0.1 of a percentage point, or 0.2 of a percentage point, or 0.3 of a percentage point, or 0.4 of a percentage point, or 0.5 of a percentage point, or 0.6 of a percentage point, or 0.7 of a percentage point, or 0.8 of a percentage point, or 0.9 of a percentage point, or one percentage point. In still other embodiments, the method may reduce the level of HbAlC in a subject in need thereof to less than 7%. In other embodiments, the level of HbAlC may be reduced to a level between 5 and 6.5%.

In another aspect, the invention provides methods of increasing glucose-dependent insulin secretion by administering to a subject a GLP1R agonist, such as OAD1, OAD2, or a pharmaceutically acceptable salt thereof, in combination with metformin, according to any of the embodiments described in the foregoing sections.

In another aspect, the invention provides methods of suppressing glucagon secretion by administering to a subject a GLP1R agonist, such as OAD1, OAD2, or a pharmaceutically acceptable salt thereof, in combination with metformin, according to any of the embodiments described in the foregoing sections.

In another aspect, the invention provides methods of treating an eating disorder by administering to a subject a GLP1R agonist, such as OAD1, OAD2, or a pharmaceutically acceptable salt thereof, in combination with metformin, according to any of the embodiments described in the foregoing sections.

In another aspect, the invention provides methods of modulating a human GLP1 receptor by administering to a subject a GLP1R agonist, such as OAD1, OAD2, or a pharmaceutically acceptable salt thereof, in combination with metformin, according to any of the embodiments described in the foregoing sections.

### Metformin Salts

In another aspect, the invention provides a salt between a GLP1R agonist, such as OAD1, OAD2, and metformin, where the GLP1R agonist activator has at least one acidic group, such as, for example, a -CO₂H group. In general, the stoichiometric ratio between metformin and the GLP1R agonist is 1:1. The invention does not require any particular amount of the salt to be present; a single pairing between a GLP1R agonist and a metformin counter ion is sufficient. Larger quantities of the salt can be present, however. For example, in some embodiments, at least 5%, or at least 10%, or at least 20%, or at least 40%, or at least 60%, or at least 80%, or at least 90%, or at least 95% of the GLP1 agonist (e.g., OAD1 or OAD2) is present in a composition as a salt with metformin (based on the total number of moles of said GLP1R agonist present in the composition). In some further embodiments, at least 5%, or at least 10%, or at least 20%, or at least 40%, or at least 60%, or at least 80%, or at least 90%, or at least 95% of the metformin is present in a composition as a salt with a GLP1R agonist (e.g., OAD1 or OAD2) (based on the total number of moles of metformin present in the composition). The salts between a GLP1R agonist and metformin need not have any particular crystalline structure or degree of crystallinity.

In an embodiment, the invention provides a salt of (S)-3-(4'-cyano-biphenyl-4-yl)-2-{[(3R,7S)-3-[4-(3,4-dichloro-benzyloxy)-phenyl]-1-methyl-2-oxo-6-((S)-1-phenyl-propyl)-2,3,5,6,7,8-hexahydro-1*H*-4-oxa-1,6-diaza-anthracene-7-carbonyl]-amino}-propionic acid and 1,1-dimethylbiguanide. Thus, an embodiment is a salt comprising a cation of 1,1-dimethylbiguanide and an anion of a GLP1R agonist, wherein the GLP1R agonist is S)-3-(4'-cyano-biphenyl-4-yl)-2-{[(3R,7S)-3-[4-(3,4-dichloro-benzyloxy)-phenyl]-1-methyl-2-oxo-6-((S)-1-phenyl-propyl)-2,3,5,6,7,8-hexahydro-1*H*-4-oxa-1,6-diaza-anthracene-7-carbonyl]-amino}-propionic acid.

In an embodiment, the invention provides a salt of (S)-2-{[(3S,8S)-3-[4-(3,4-dichloro-benzyloxy)-phenyl]-7-((S)-1-phenyl-propyl)-2,3,6,7,8,9-hexahydro-[1,4]dioxino[2,3-g]isoquinoline-8-carbonyl]-amino}-3-[4-(2,3-dimethyl-pyridin-4-yl)-phenyl]-propionic acid and 1,1-dimethylbiguanide. Thus, an embodiment is a salt comprising a cation of 1,1-dimethylbiguanide and an anion of a GLP1R agonist, wherein the GLP1R agonist is (S)-2-{[(3S,8S)-3-[4-(3,4-dichloro-benzyloxy)-phenyl]-7-((S)-1-phenyl-propyl)-2,3,6,7,8,9-hexahydro-[1,4]dioxino[2,3-g]isoquinoline-8-carbonyl]-amino}-3-[4-(2,3-dimethyl-pyridin-4-yl)-phenyl]-propionic acid.

In some embodiments, the invention provides a pharmaceutical composition comprising a metformin salt of a GLP1R agonist, or OAD1 or OAD2, and further comprising a pharmaceutically acceptable carrier, diluent, or excipient, or mixtures thereof. In some such embodiments, the pharmaceutical composition may also comprise additional quantities of metformin and/or a GLP1R agonist.

In any of the aforementioned methods of treatment, administration of metformin and a GLP1R agonist may be carried out, in whole or in part, by administering a metformin salt of a GLP1R agonist.

### EXAMPLES

### Example 1: Preparation of Metformin Free Base

Metformin free base is prepared via a modification of Bohuon, US Patent 4,080,472. A column of 200 mL of Dowex 1-X8 (Cl form) is packed as a slurry in water and eluted with one column volume of water, one column volume of 1 N NaOH, and then eluted with water until the eluent is at pH ~7. Metformin HCl salt (33 g) is taken up into 100 mL of 4:1 methanol: water with mild heat and is added to the column and eluted with four column volumes of water. The eluent fractions containing metformin are combined and concentrated in vacuo to an oil. The oil is taken up in 100 mL of 1:1 toluene:methanol and filtered. The filtrate was concentrated in vacuo to afford 26 g of metformin free base as an oil which solidifies after storage at high vacuum.

### Example 2: Formulation of OAD1 hydrochloride ("Compound 1")

Compound 1 (179 mg) was suspended in 4 mL of 10% aqueous polysorbate 80 (Tween 80, Fisher L#100938). The suspension was sonicated for five min, then was homogenized for one minute at low speed until a uniform suspension was obtained.
Hydroxypropylmethylcellulose E3 (HPMC E3, Dow Chemical L#YF290124L1) (160 mg) was added to the suspension with vortex mixing. The volume was adjusted to 80 mL with deionized water and the mixture was stirred magnetically for 5 min.

This mixture constitutes 2 mg/mL of Compound 1 in 0.2% HPMC E3, 0.5% Tween 80 in water.

Example 2A: Ten mL of the above mixture of 2 mg/mL Compound 1 in 0.2% HPMC E3, 0.5% Tween 80 in water constitutes Example 2A.

Example 2B: Ten mL of the above mixture of 2 mg/mL Compound 1 in 0.2% HPMC E3, 0.5% Tween 80 in water was treated with 80 mg of metformin hydrochloride and mixed using magnetic stirrer for 5 minutes, to constitute Example 2B.

Example 2C: Ten mL of the above mixture of 2 mg/mL Compound 1 in 0.2% HPMC E3, 0.5% Tween 80 in water was treated with 79 mg of metformin free base (from Example 1) and mixed using magnetic stirrer for 5 minutes, to constitute Example 2C.

### Example 3: Oral Exposure in Mouse

The compositions of Examples 2A and 2B and 2C were analyzed for in vivo bioavailability using female C57BL/6 mice, n = 3, weighing 20-25 g. The dose (5 mL/kg of each of 2A, 2B, and 2C, which is the equivalent of 10 mg/kg of Compound 1) was administered orally to animals in the fed state. The compositions were administered by oral gavage with a 22 gauge gavage needle attached to a syringe. Following dosing, blood samples for pharmacokinetic evaluation were collected via tail nick, in duplicates from each animal at 0.5, 1, 1.5, 2, and 4 hr post dosing. After each time point, all blood samples were collected into tubes containing 100µL acetonitrile, processed, and cooled in a refrigerator (2 to 8 °C).

The concentrations of Compound 1 in mouse plasma were determined by a LC-MS/MS assay following a protein precipitation step with acetonitrile. Pharmacokinetic analysis was performed using the WINNONLIN software program (Pharsight, Inc. Mountain View, Calif.). The area under the plasma concentration-time curve (AUC₀₋ₜ) is calculated from the first time point (0 min) up to the last time point, which was 4 hours. The Cₘₐₓ is calculated from the maximum of the fitted curve, and Tₘₐₓ is the time at which Cₘₐₓ occurs. The results of analysis of the formulations of Examples 2A, 2B, and 2C are shown in Table 1.

**Table 1 - Oral Exposure in Mouse**

| | Formulation of Example 2A | Formulation of Example 2B | Formulation of Example |
|---|---|---|---|
| | Mean (SD) | Mean (SD) | 2C Mean (SD) |
| Tₘₐₓ (h) | 1.2 (0.3) | 1.0 (0.0) | 1.3 (0.3) |
| Cₘₐₓ (ng/mL) | 55 (18) | 208 (135) | 310 (280) |
| AUC₀₋₄ (h*ng/mL) | 124 (22) | 413 (170) | 595 (500) |

An exact test appropriate for small samples was performed on the calculated parameters: Cₘₐₓ and AUC₀₋₄. Sample 1 is the data from the formulation of Example 2A and sample 2 is the pooled data from the formulations of Examples 2B and 2C. The Mann-Whitney-Wilcoxon test is a test of the equivalence of two distributions focused on detecting differences in location (average values). The exact test is valid for sample sizes as small as 3 in each group. The test is a rank test, and is based on the number of order pairs (one value from sample 1 and one value from sample 2). Under the hypothesis of no difference, it is expected that half of the ordered pairs would have the value from the first sample smaller than the value from the second sample. For the parameters Cₘₐₓ and AUC₀₋₄, the observation was made that the largest value in sample 1 is smaller than the smallest value in sample 2. In this case, with sample sizes of 3 and 6 (pooling the data from the formulations of Examples 2B and 2C), the exact test p-value had nominal statistical significance p<0.05 (exact 2-sided p=0.02).

## Claims

1. A combination comprising a GLP1R agonist in combination with metformin for treating type 2 diabetes, for lowering blood-glucose in a subject, for increasing the therapeutic effectiveness of metformin, for increasing the therapeutic effectiveness of a glucagon-like peptide 1 receptor agonist ("GLP1R agonist"), for increasing the oral bioavailability of a GLP1R agonist, for use in treating a condition selected from the group consisting of metabolic syndrome, glucose intolerance, hyperglycemia, dyslipidemia, hypertriglyceridemia, syndrome X, insulin resistance, impaired glucose tolerance (IGT), obesity, diabetic dyslipidemia, hyperlipidemia, arteriosclerosis, atherosclerosis, other cardiovascular diseases, hypertension, metabolic disorders where agonism of GLP1R is beneficial, or complications resulting from or associated with diabetes, for treating type 1 diabetes, for treating obesity, for slowing gastric emptying, for lowering an HbA1c levels, for increasing glucose-dependent insulin secretion, for suppressing glucagon secretion, for treating an eating disorder, or for modulating a human GLP1R receptor, wherein the GLP1R agonist is administered orally and is (S)-3-(4'-cyano-biphenyl-4-yl)-2-{[(3R,7S)-3-[4-(3,4-dichloro-benzyloxy)-phenyl]-1-methyl-2-oxo-6-((S)-1-phenyl-propyl)-2,3,5,6,7,8-hexahydro-1*H*-4-oxa-1,6-diaza-anthracene-7-carbonyl]-amino}-propionic acid or a pharmaceutically acceptable salt thereof, or (S)-2-{[(3S,8S)-3-[4-(3,4-dichloro-benzyloxy)-phenyl]-7-((S)-1-phenyl-propyl)-2,3,6,7,8,9-hexahydro-[1,4]dioxino[2,3-g]isoquinoline-8-carbonyl]-amino}-3-[4-(2,3-dimethyl-pyridin-4-yl)-phenyl]-propionic acid or a pharmaceutically acceptable salt thereof.

2. The combination for the use of claim 1, wherein the metformin is metformin hydrochloride or 1,1-dimethylbiguanide.

3. The combination for the use of any of the previous claims, wherein the GLP1R agonist has an absolute bioavailability after oral administration to a subject of at least 1%.

4. The combination for the use of any of the previous claims, wherein the GLP1R agonist is (S)-3-(4'-cyano-biphenyl-4-yl)-2-{[(3R,7S)-3-[4-(3,4-dichloro-benzyloxy)-phenyl]-1-methyl-2-oxo-6-((S)-1-phenyl-propyl)-2,3,5,6,7,8-hexahydro-1*H*-4-oxa-1,6-diaza-anthracene-7-carbonyl]-amino}-propionic acid or a pharmaceutically acceptable salt thereof.

5. The combination for the use of any one of claims 1 to 3, wherein the GLP1R agonist is (S)-2-{[(3S,8S)-3-[4-(3,4-dichloro-benzyloxy)-phenyl]-7-((S)-1-phenyl-propyl)-2,3,6,7,8,9-hexahydro-[1,4]dioxino[2,3-g]isoquinoline-8-carbonyl]-amino}-3-[4-(2,3-dimethyl-pyridin-4-yl)-phenyl]-propionic acid or a pharmaceutically acceptable salt thereof.

6. The combination for the use of any of the previous claims, wherein the GLP1R agonist and the metformin are administered to the subject simultaneously, or wherein the GLP1R agonist and the metformin are administered, such that one is administered subsequent to the other.

7. The combination for the use of any of the previous claims, wherein the GLP1R agonist and the metformin are both administered orally in the same dosage form.

8. The combination for the use of any of the previous claims, wherein the subject is a human.

9. The combination for the use of any of the previous claims, wherein the amount of GLP1R agonist is between 1 mg/day and 1000 mg/day, and wherein the amount of metformin is between 250 mg/day and 2500 mg/day.

10. The combination for the use of any one of claims 1 to 8, wherein the GLP1R agonist is administered in a suboptimal amount, or wherein the metformin is administered in a suboptimal amount, or wherein the GLP1R agonist and the metformin are both administered in suboptimal amounts.

11. A pharmaceutical composition comprising a glucagon-like peptide 1 receptor ("GLP1R agonist") agonist, metformin, and at least one pharmaceutically acceptable carrier, wherein the GLP1R agonist is (S)-3-(4'-cyano-biphenyl-4-yl)-2-{[(3R,7S)-3-[4-(3,4-dichloro-benzyloxy)-phenyl]-1-methyl-2-oxo-6-((S)-1-phenyl-propyl)-2,3,5,6,7,8-hexahydro-1*H*-4-oxa-1,6-diaza-anthracene-7-carbonyl]-amino}-propionic acid or a pharmaceutically acceptable salt thereof, or (S)-2-{[(3S,8S)-3-[4-(3,4-dichloro-benzyloxy)-phenyl]-7-((S)-1-phenyl-propyl)-2,3,6,7,8,9-hexahydro-[1,4]dioxino[2,3-g]isoquinoline-8-carbonyl]-amino}-3-[4-(2,3-dimethyl-pyridin-4-yl)-phenyl]-propionic acid or a pharmaceutically acceptable salt thereof.

12. The pharmaceutical composition of claim 11, wherein the GLP1R agonist is (S)-3-(4'-cyano-biphenyl-4-yl)-2-{[(3R,7S)-3-[4-(3,4-dichloro-benzyloxy)-phenyl]-1-methyl-2-oxo-6-((S)-1-phenyl-propyl)-2,3,5,6,7,8-hexahydro-1*H*-4-oxa-1,6-diaza-anthracene-7-carbonyl]-amino}-propionic acid or a pharmaceutically acceptable salt thereof.

13. The pharmaceutical composition of claim 11, wherein the GLP1R agonist is (S)-2-{[(3S,8S)-3-[4-(3,4-dichloro-benzyloxy)-phenyl]-7-((S)-1-phenyl-propyl)-2,3,6,7,8,9-hexahydro-[1,4]dioxino[2,3-g]isoquinoline-8-carbonyl]-amino}-3-[4-(2,3-dimethyl-pyridin-4-yl)-phenyl]-propionic acid or a pharmaceutically acceptable salt thereof.

14. The pharmaceutical composition of any one of claims 11 to 13, wherein the metformin is metformin hydrochloride or 1,1-dimethylbiguanide.

15. The pharmaceutical composition of any one of claims 11 or 13, wherein the GLP1R agonist has an absolute oral bioavailability of greater than 1%.

16. The pharmaceutical composition of claim 11, wherein the GLP1R agonist is (S)-3-(4'-cyano-biphenyl-4-yl)-2-{[(3R,7S)-3-[4-(3,4-dichloro-benzyloxy)-phenyl]-1-methyl-2-oxo-6-((S)-1-phenyl-propyl)-2,3,5,6,7,8-hexahydro-1*H-*4-oxa-1,6-diaza-anthracene-7-carbonyl]-amino}-propionic acid or a pharmaceutically acceptable salt thereof;
wherein the amount of the GLP1R agonist is less than 250 mg;
wherein the amount of metformin is between 100 mg and 1000 mg;
wherein the weight to weight ratio of the GLP1 agonist to metformin is between 1:2 and 1:100,
optionally wherein the GLP1R agonist is (S)-3-(4'-cyano-biphenyl-4-yl)-2-{[(3R,7S)-3-[4-(3,4-dichloro-benzyloxy)-phenyl]-1-methyl-2-oxo-6-((S)-1-phenyl-propyl)-2,3,5,6,7,8-hexahydro-1*H*-4-oxa-1,6-diaza-anthracene-7-carbonyl]-amino}-propionicacid hydrochloride salt, and
optionally wherein the metformin is metformin hydrochloride or 1,1-dimethylbiguanide, and
optionally wherein the weight to weight ratio of the GLP1 agonist to metformin is between 1:2 and 1:20.

## Patentansprüche

1. Kombination, umfassend einen GLP1R-Agonisten in Kombination mit Metformin zum Behandeln von Diabetes Typ 2, zum Senken des Blutzuckerspiegels bei einem Patienten, zum Erhöhen der therapeutischen Wirksamkeit von Metformin, zum Erhöhen der therapeutischen Wirksamkeit eines Agonisten des Glucagon-ähnlichen Peptid-1-Rezeptors ("GLP1R-Agonist"), zum Erhöhen der oralen Bioverfügbarkeit eines GLP1R-Agonisten, zur Verwendung bei der Behandlung eines Zustands, der aus der Gruppe ausgewählt ist, bestehend aus metabolischem Syndrom, Glucoseintoleranz, Hyperglykämie, Dyslipidämie, Hypertriglyceridämie, Syndrom X, Insulinresistenz, beeinträchtigter Glucosetoleranz (IGT), Fettleibigkeit, diabetischer Dyslipidämie, Hyperlipidämie, Arteriosklerose, Atherosklerose, anderen kardiovaskulären Erkrankungen, Bluthochdruck, Stoffwechselstörungen, bei denen Agonismus von GLP1R vorteilhaft ist, oder Komplikationen, die sich aus Diabetes ergeben oder damit verbunden sind, zum Behandeln von Diabetes Typ 1, zum Behandeln von Fettleibigkeit, zum Verlangsamen der Magenentleerung, zum Senken von HbA1c-Werten, zum Erhöhen der glucoseabhängigen Insulinsekretion, zum Unterdrücken der Glucagonsekretion, zum Behandeln einer Essstörung oder zum Modulieren eines humanen GLP1R-Rezeptors, wobei der GLP1R-Agonist oral verabreicht wird und (S)-3-(4'-Cyano-biphenyl-4-yl)-2-{[(3R,7S)-3-[4-(3,4-dichlor-benzyloxy)-phenyl]-1-methyl-2-oxo-6-((S) -1-phenyl-propyl)-2,3,5,6,7,8-hexahydro-1*H*-4-oxa-1,6-diaza-anthracen-7-carbonyl]-amino}-propionsäur e oder ein pharmazeutisch annehmbares Salz davon, oder (S)-2-{[(3S,8S)-3-[4-(3,4-Dichlor-benzyloxy)-phenyl]-7-((S)-1-phenyl-propyl)-2,3,6,7,8,9-hexahydro-[1, 4]dioxino[2,3-g]isoquinolin-8-carbonyl]-amino}-3-[4-(2,3-dimethyl-pyridin-4-yl)-phenyl]-propionsäure oder ein pharmazeutisch annehmbares Salz davon ist.

2. Kombination zur Verwendung nach Anspruch 1, wobei das Metformin Metforminhydrochlorid oder 1,1-Dimethylbiguanid ist.

3. Kombination zur Verwendung nach einem der vorhergehenden Ansprüche, wobei der GLP1R-Agonist nach der oralen Verabreichung an einen Patienten eine absolute Bioverfügbarkeit von mindestens 1 % aufweist.

4. Kombination zur Verwendung nach einem der vorhergehenden Ansprüche, wobei der GLP1 R-Agonist (S)-3-(4'-Cyano-biphenyl-4-yl)-2-{[(3R,7S)-3-[4-(3,4-dichlor-benzyloxy)-phenyl]-1-methyl-2-oxo-6-((S) -1-phenyl-propyl)-2,3,5,6,7,8-hexahydro-1*H*-4-oxa-1,6-diaza-anthracen-7-carbonyl]-amino}-propionsäur e oder ein pharmazeutisch annehmbares Salz davon ist.

5. Kombination zur Verwendung nach einem der Ansprüche 1 bis 3, wobei der GLP1R-Agonist (S)-2-{[(3S,8S)-3-[4-(3,4-Dichlor-benzyloxy)-phenyl]-7-((S)-1-phenyl-propyl)-2,3,6,7,8,9-hexahydro-[1, 4]dioxino[2,3-g]isoquinolin-8-carbonyl]-amino}-3-[4-(2,3-dimethyl-pyridin-4-yl)-phenyl]-propionsäure oder ein pharmazeutisch annehmbares Salz davon ist.

6. Kombination zur Verwendung nach einem der vorhergehenden Ansprüche, wobei der GLP1R-Agonist und das Metformin dem Patienten simultan verabreicht werden oder wobei der GLP1R-Agonist und das Metformin derart verabreicht werden, dass eines im Anschluss an das andere verabreicht wird.

7. Kombination zur Verwendung nach einem der vorhergehenden Ansprüche, wobei der GLP1R-Agonist und das Metformin beide oral in der gleichen Dosierungsform verabreicht werden.

8. Kombination zur Verwendung nach einem der vorhergehenden Ansprüche, wobei der Patient ein Mensch ist.

9. Kombination zur Verwendung nach einem der vorhergehenden Ansprüche, wobei die Menge an GLP1R-Agonist zwischen 1 mg/Tag und 1000 mg/Tag beträgt und wobei die Menge an Metformin zwischen 250 mg/Tag und 2500 mg/Tag beträgt.

10. Kombination zur Verwendung nach einem der Ansprüche 1 bis 8, wobei der GLP1R-Agonist in einer suboptimalen Menge verabreicht wird oder wobei das Metformin in einer suboptimalen Menge verabreicht wird oder wobei der GLP1R-Agonist und das Metformin beide in suboptimalen Mengen verabreicht werden.

11. Pharmazeutische Zusammensetzung, umfassend einen Agonisten des Glucagon-ähnlichen Peptid-1-Rezeptors ("GLP1R-Agonist"), Metformin und zumindest einen pharmazeutisch annehmbaren Träger, wobei der GLP1R-Agonist (S)-3-(4'-Cyano-biphenyl-4-yl)-2-{[(3R,7S)-3-[4-(3,4-dichlor-benzyloxy)-phenyl]-1-methyl-2-oxo-6-((S) -1-phenyl-propyl)-2,3,5,6,7,8-hexahydro-1*H*-4-oxa-1,6-diaza-anthracen-7-carbonyl]-amino}-propionsäur e oder ein pharmazeutisch annehmbares Salz davon, oder (S)-2-{[(3S,8S)-3-[4-(3,4-Dichlor-benzyloxy)-phenyl]-7-((S)-1-phenyl-propyl)-2,3,6,7,8,9-hexahydro-[1, 4]dioxino[2,3-g]isoquinolin-8-carbonyl]-amino}-3-[4-(2,3-dimethyl-pyridin-4-yl)-phenyl]-propionsäure oder ein pharmazeutisch annehmbares Salz davon ist.

12. Pharmazeutische Zusammensetzung nach Anspruch 11, wobei der GLP1R-Agonist (S)-3-(4'-Cyano-biphenyl-4-yl)-2-{[(3R,7S)-3-[4-(3,4-dichlor-benzyloxy)-phenyl]-1-methyl-2-oxo-6-((S) -1-phenyl-propyl)-2,3,5,6,7,8-hexahydro-1H-4-oxa-1,6-diaza-anthracen-7-carbonyl]-amino}-propionsäur e oder ein pharmazeutisch annehmbares Salz davon ist.

13. Pharmazeutische Zusammensetzung nach Anspruch 11, wobei der GLP1R-Agonist (S)-2-{[(3S,8S)-3-[4-(3,4-Dichlor-benzyloxy)-phenyl]-7-((S)-1-phenyl-propyl)-2,3,6,7,8,9-hexahydro-[1, 4]dioxino[2,3-g]isoquinolin-8-carbonyl]-amino}-3-[4-(2,3-dimethyl-pyridin-4-yl)-phenyl]-propionsäure oder ein pharmazeutisch annehmbares Salz davon ist.

14. Pharmazeutische Zusammensetzung nach einem der Ansprüche 11 bis 13, wobei das Metformin Metforminhydrochlorid oder 1,1-Dimethylbiguanid ist.

15. Pharmazeutische Zusammensetzung nach einem der Ansprüche 11 oder 13, wobei der GLP1R-Agonist eine absolute orale Bioverfügbarkeit von mehr als 1 % aufweist.

16. Pharmazeutische Zusammensetzung nach Anspruch 11, wobei der GLP1R-Agonist (S)-3-(4'-Cyano-biphenyl-4-yl)-2-{[(3R,7S)-3-[4-(3,4-dichlor-benzyloxy)-phenyl]-1-methyl-2-oxo-6-((S) -1-phenyl-propyl)-2,3,5,6,7,8-hexahydro-1*H*-4-oxa-1,6-diaza-anthracen-7-carbonyl]-amino}-propionsäur e oder ein pharmazeutisch annehmbares Salz davon ist;
wobei die Menge des GLP1R-Agonisten weniger als 250 mg beträgt;
wobei die Menge an Metformin zwischen 100 mg und 1000 mg beträgt;
wobei das Gewichts-Gewichts-Verhältnis des GLP1-Agonisten zu Metformin zwischen 1:2 und 1:100 beträgt, wobei optional der GLP1R-Agonist (S)-3-(4'-Cyano-biphenyl-4-yl)-2-{[(3R,7S)-3-[4-(3,4-dichlor-benzyloxy)-phenyl]-1-methyl-2-oxo-6-((S) -1-phenyl-propyl)-2,3,5,6,7,8-hexahydro-1*H*-4-oxa-1,6-diaza-anthracen-7-carbonyl]-amino}-propionsäur ehydrochloridsalz ist, und wobei optional das Metformin Metforminhydrochlorid oder 1,1-Dimethylbiguanid ist, und wobei optional das Gewichts-Gewichts-Verhältnis des GLP1-Agonisten zu Metformin zwischen 1:2 und 1:20 beträgt.

## Revendications

1. Combinaison comprenant un agoniste du GLP1R en combinaison avec de la metformine pour traiter le diabète de type 2, pour abaisser la glycémie chez un sujet, pour augmenter l'efficacité thérapeutique de la metformine, pour augmenter l'efficacité thérapeutique d'un agoniste du récepteur du peptide 1 de type glucagon (« agoniste du GLP1R »), pour augmenter la biodisponibilité orale d'un agoniste du GLP1R, pour utilisation dans le traitement d'un état choisi dans le groupe constitué par un syndrome métabolique, l'intolérance au glucose, l'hyperglycémie, la dyslipidémie, l'hypertriglycéridémie, le syndrome X, l'insulino-résistance, la tolérance au glucose altérée (IGT), l'obésité, la dyslipidémie diabétique, l'hyperlipidémie, l'artériosclérose, l'athérosclérose, d'autres maladies cardiovasculaires, l'hypertension, les troubles métaboliques dans lesquels l'agonisme du GLP1R est bénéfique, ou des complications résultant du diabète ou associées à celui-ci, pour traiter le diabète de type 1, pour traiter l'obésité, pour ralentir la vidange gastrique, pour abaisser les taux de HbAlc, pour augmenter la sécrétion d'insuline dépendant du glucose, pour supprimer la sécrétion de glucagon, pour traiter un trouble alimentaire, ou pour moduler un récepteur GLP1R humain, ledit agoniste du GLP1R étant administré par voie orale et étant l'acide (S)-3-(4'-cyano-biphényl-4-yl)-2-{[(3R,7S)-3-[4-(3,4-dichloro-benzyloxy)-phényl]-1-méthyl-2-o xo-6-((S)-1-phényl-propyl)-2,3,5,6,7,8-hexahydro-1*H*-4-oxa-1,6-diaza-anthracène-7-carbonyl]-a mino}-propionique ou un sel pharmaceutiquement acceptable de celui-ci, ou l'acide (S)-2-{[(3S,8S)-3-[4-(3,4-dichloro-benzyloxy)-phényl]-7-((S)-1-phényl-propyl)-2,3,6,7,8,9-hexa hydro-[1,4]dioxino[2,3-g]isoquinoline-8-carbonyl]-amino}-3-[4-(2,3-diméthyl-pyridin-4-yl)-phé nyl]-propionique ou un sel pharmaceutiquement acceptable de celui-ci.

2. Combinaison pour utilisation selon la revendication 1, ladite metformine étant le chlorhydrate de metformine ou le 1,1-diméthylbiguanide.

3. Combinaison pour utilisation selon l'une quelconque des revendications précédentes, ledit agoniste du GLP1R présentant une biodisponibilité absolue après administration orale à un sujet supérieure ou égale à 1 %.

4. Combinaison pour utilisation selon l'une quelconque des revendications précédentes, ledit agoniste du GLP1R étant l'acide (S)-3-(4'-cyano-biphényl-4-yl)-2-{[(3R,7S)-3-[4-(3,4-dichloro-benzyloxy)-phényl]-1-méthyl-2-o xo-6-((S)-1-phényl-propyl)-2,3,5,6,7,8-hexahydro-1*H-*4-oxa-1,6-diaza-anthracène-7-carbonyl]-a mino}-propionique ou un sel pharmaceutiquement acceptable de celui-ci.

5. Combinaison pour utilisation selon l'une quelconque des revendications 1 à 3, ledit agoniste du GLP1R étant l'acide (S)-2-{[(3S,8S)-3-[4-(3,4-dichloro-benzyloxy)-phényl]-7-((S)-1-phényl-propyl)-2,3,6,7,8,9-hexa hydro-[1,4]dioxino[2,3-g]isoquinoline-8-carbonyl]-amino}-3-[4-(2,3-diméthyl-pyridin-4-yl)-phé nyl]-propionique ou un sel pharmaceutiquement acceptable de celui-ci.

6. Combinaison pour utilisation selon l'une quelconque des revendications précédentes, ledit agoniste du GLP1R et ladite metformine étant administrés au sujet en même temps, ou ledit agoniste du GLP1R et ladite metformine étant administrés de sorte que l'un soit administré après l'autre.

7. Combinaison pour utilisation selon l'une quelconque des revendications précédentes, ledit agoniste du GLP1R et ladite metformine étant tous les deux administrés par voie orale sous la même forme posologique.

8. Combinaison pour utilisation selon l'une quelconque des revendications précédentes, ledit sujet étant un humain.

9. Combinaison pour utilisation selon l'une quelconque des revendications précédentes, ladite quantité de l'agoniste du GLP1R étant comprise entre 1 mg/jour et 1000 mg/jour, et ladite quantité de metformine étant comprise entre 250 mg/jour et 2500 mg/jour.

10. Combinaison pour utilisation selon l'une quelconque des revendications 1 à 8, ledit agoniste du GLP1R étant administré en une quantité sous-optimale, ou ladite metformine étant administrée en une quantité sous-optimale, ou ledit agoniste du GLP1R et ladite metformine étant tous les deux administrés en des quantités sous-optimales.

11. Composition pharmaceutique comprenant un agoniste du récepteur du peptide 1 de type glucagon (« agoniste du GLP1R »), de la metformine et au moins un excipient pharmaceutiquement acceptable, ledit agoniste du GLP1R étant l'acide (S)-3-(4'-cyano-biphényl-4-yl)-2-{[(3R,7S)-3-[4-(3,4-dichloro-benzyloxy)-phényl]-1-méthyl-2-o xo-6-((S)-1-phényl-propyl)-2,3,5,6,7,8-hexahydro-1*H*-4-oxa-1,6-diaza-anthracène-7-carbonyl]-a mino}-propionique ou un sel pharmaceutiquement acceptable de celui-ci, ou l'acide (S)-2-{[(3S,8S)-3-[4-(3,4-dichloro-benzyloxy)-phényl]-7-((S)-1-phényl-propyl)-2,3,6,7,8,9-hexa hydro-[1,4]dioxino[2,3-g]isoquinoline-8-carbonyl]-amino}-3-[4-(2,3-diméthyl-pyridin-4-yl)-phé nyl]-propionique ou un sel pharmaceutiquement acceptable de celui-ci.

12. Composition pharmaceutique selon la revendication 11, ledit agoniste du GLP1R étant l'acide (S)-3-(4'-cyano-biphényl-4-yl)-2-{[(3R,7S)-3-[4-(3,4-dichloro-benzyloxy)-phényl]-1-méthyl-2-o xo-6-((S)-1-phényl-propyl)-2,3,5,6,7,8-hexahydro-1*H*-4-oxa-1,6-diaza-anthracène-7-carbonyl]-a mino}-propionique ou un sel pharmaceutiquement acceptable de celui-ci.

13. Composition pharmaceutique selon la revendication 11, ledit agoniste du GLP1R étant l'acide (S)-2-{[(3S,8S)-3-[4-(3,4-dichloro-benzyloxy)-phényl]-7-((S)-1-phényl-propyl)-2,3,6,7,8,9-hexa hydro-[1,4]dioxino[2,3-g]isoquinoline-8-carbonyl]-amino}-3-[4-(2,3-diméthyl-pyridin-4-yl)-phé nyl]-propionique ou un sel pharmaceutiquement acceptable de celui-ci.

14. Combinaison pharmaceutique pour utilisation selon l'une quelconque des revendications 11 à 13, ladite metformine étant le chlorhydrate de metformine ou le 1,1-diméthylbiguanide.

15. Composition pharmaceutique selon l'une quelconque des revendications 11 ou 13, ledit agoniste du GLP1R présentant une biodisponibilité orale absolue supérieure à 1 %.

16. Composition pharmaceutique selon la revendication 11, ledit agoniste du GLP1R étant l'acide (S)-3-(4'-cyano-biphényl-4-yl)-2-{[(3R,7S)-3-[4-(3,4-dichloro-benzyloxy)-phényl]-1-méthyl-2-o xo-6-((S)-1-phényl-propyl)-2,3,5,6,7,8-hexahydro-1*H*-4-oxa-1,6-diaza-anthracène-7-carbonyl]-a mino}-propionique ou un sel pharmaceutiquement acceptable de celui-ci ;
ladite quantité de l'agoniste du GLP1R étant inférieure à 250 mg ;
ladite quantité de la metformine étant comprise entre 100 mg et 1000 mg ;
le rapport en poids par poids de l'agoniste du GLP1 sur la metformine étant compris entre 1:2 et 1:100,
éventuellement ledit agoniste du GLP1R étant le sel de chlorhydrate de l'acide (S)-3-(4'-cyano-biphényl-4-yl)-2-{[(3R,7S)-3-[4-(3,4-dichloro-benzyloxy)-phényl]-1-méthyl-2-o xo-6-((S)-1phényl-propyl)-2,3,5,6,7,8-hexahydro-1*H*-4-oxa-1,6-diaza-anthracène-7-carbonyl]-a mino}-propionique, et
éventuellement ladite metformine étant le chlorhydrate de metformine ou le 1,1-diméthylbiguanide, et
éventuellement ledit rapport en poids par poids de l'agoniste du GLP1 sur la metformine étant compris entre 1:2 et 1:20.
